# EUROPEAN PATENT SPECIFICATION

(11) **EP 1 443 928 B1**
(45) Date of publication and mention of the grant of the patent: **27.07.2011**
(21) Application number: 02778601.1
(22) Date of filing: 16.10.2002
(51) Int. Cl.: A61K 31/47, A61K 31/166, A61K 31/167, A61K 31/19, A61K 31/4406, A61K 31/4709, A61P 35/00

(54) **TREATMENT OF NEURODEGENERATIVE DISEASES AND CANCER OF THE BRAIN**
BEHANDLUNG VON NEURODEGENERATIVEN ERKRANKUNGEN UND KREBS IM GEHIRN
TRAITEMENT DES MALADIES NEURODEGENERATIVES ET DU CANCER DU CERVEAU

(30) Priority: 16.10.2001 US 329705 P
(43) Date of publication of application: 11.08.2004
(60) Divisional application: 10183856.3
(73) Proprietor: Sloan-Kettering Institute For Cancer Research, New York, New York 10021 (US)
(72) Inventor: RICHON, Victoria, M., Rye, NY 10580 (US); MARKS, Paul, A., Washington, CT 06793 (US); RIFKIND, Richard, A., New York, NY 10022 (US)
(74) Representative: MacLean, Martin Robert
(86) International application number: PCT/US2002/033246
(87) International publication number: WO 2003/032921

(56) References cited:
- EP-A1- 0 931 792
- WO-A-98/39965
- WO-A-98/55449
- WO-A2-00/08048
- US-A- 5 055 608
- US-A- 5 369 108
- US-A- 5 773 474
- PAUL A. MARKS ET AL.: "Histone deacetylase inhibitors: Inducers of differentiation or apoptosis of transformed cells" JOURNAL OF THE NATIONAL CANCER INSTITUTE, vol. 92, no. 15, 2 August 2000 (2000-08-02), pages 1210-1216, XP002422972

## Description

Compounds which inhibit histone deacetylases (HDACs) have been shown to cause growth arrest, differentiation and/or apoptosis of many different types of tumor cells *in vitro* and *in vivo*. HDACs catalyze the removal of the acetyl group from the lysine residues in the N-terminal tails of nucleosomal core histones resulting in a more compact chromatin structure, a configuration that is generally associated with repression of transcription. These HDAC inhibitors fall into four general classes: 1) short-chain fatty acids (*e.g.*, 4-phenylbutyrabe and valproic acid); hydroxamic acids (*e.g.*, SAHA, Pyroxamide, CBHA, trichostatin A (TSA), oxamflatin and CHAPs such as, CHAP1 and CHAP 31); 3) cyclic tetrapeptides (Trapoxin A and Apicidin; 4) benzamides (*e.g.*, MS-275); and other compounds such as Scriptaid. Examples of such compounds can be found in U.S. Patent Nos. 5,369,108, issued on November 29, 1994, 5,700,811, issued on December 23,1997, and 5,773,474, issued on June 30, 1998 to Breslow et al., U.S. Patent Nos. 5,055,608, issued on October 8,1991, and 5,175,191, issued on December 29,1992 to Marks et al., as well as, Yoshida, M., et al., Bioassays 17, 423-430 (1995), Saito, A., et al., PNAS USA 96, 4592-4597, (1999), Furamai R. et al., PNAS USA 98 (1), 87-92 (2001), Komatsu, Y., et al., Cancer Res. 61(11), 4459-4466 (2001), Su, G.H., et al., Cancer Res. 60, 3137-3142 (2000), Lee, B.I et al., Cancer Res. 61 (3), 931-934, Suzuki, T., et al., J. Med. Chem. 42(15), 3001-3003 (1999) and published PCT Application WHO 01/18171 published on March 15, 2001 to Sloan-Kettering Institute for Cancer Research and The Trustees of Columbia University.

Some notable hydroxamic acid based HDAC inhibitors are suberoylanilide hydroxamic acid (SAHA), m-carboxycinnamic acid bishydroxamate (CBHA) and pyroxamide. SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

WO 00/08048 describes an inhibitor of histone deacetylase.

WO 98/55449 describes hydroxamic acid compounds having anti-cancer and anti-parasitic properties.

Marks P.A. et al., J. Nat. Cancer Institute, Vol. 92(15), 2 August 2000, pages 1210-1216, reviews histone deacetylase inhibitors.

EP 0931792 describes depsipeptides containing non-natural amino acids.

WO 98/39965 describes unsaturated oxyalkylene esters and uses thereof.

US5055608, US5369108 and US5773474 describe potent inducers of terminal differentiation.

WO 02/26696 describes carbamic acid compounds comprising an amide linkage, as HDAC inhibitors.

WO 97/35990 describes histone deacetylases and uses thereof.

The invention provides the use of suberoylanilide hydroxamic acid (SAHA) represented by the following structure: 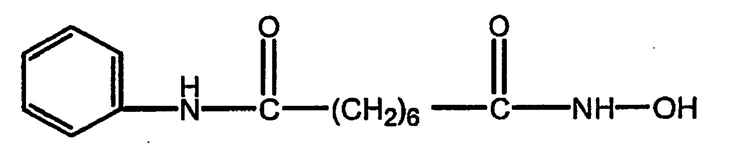 or a pharmaceutically acceptable salt or hydrate thereof, and a pharmaceutically acceptable carrier or diluent, for manufacture of an oral medicament for treating brain cancer in a mammal.

It has been surprisingly discovered that certain HDAC inhibitors, for example, SAHA, CBHA and pyroxamide can cross the blood brain barrier at sufficient amounts to significantly inhibit HDAC activity causing the accumulation of acetylated histones in the brain. This discovery indicates the use of HDAC inhibitors for inhibiting HDAC in the brain, for the treatment of disorders of the central nervous system including cancer of the brain and neurodegenerative diseases.

The present application is directed to inhibiting HDAC in the brain of a mammal. This comprises administering to an individual a HDAC inhibiting amount of a histone deacetylase inhibitor compound. The present application is further directed to treating disease of the central nervous system (CNS) comprising administering to a individual in need of treatment a therapeutically effective amount of an inhibitor of HDAC which is SAHA. The disease is cancer of the brain.

The individual is a mammal such as a primate or human.

### BRIEF DESCRIPTION OF THE DRAWINGS

FIG. 1 is a scan of a Western blot and Coomassie stained gel indicating levels of acetylated histone (αAcH3) at the indicated timepoints following treatment with vehicle (DMSO) or three doses of SAHA (100 mg/kg/hr).
FIG. 2 is a scan of a Western blot and Coomassie stained gel indicating levels of acetylated histone (αAcH4) at the indicated timepoints following treatment with vehicle (DMSO) or three doses of Pyroxamide (100 mg/kg/hr).

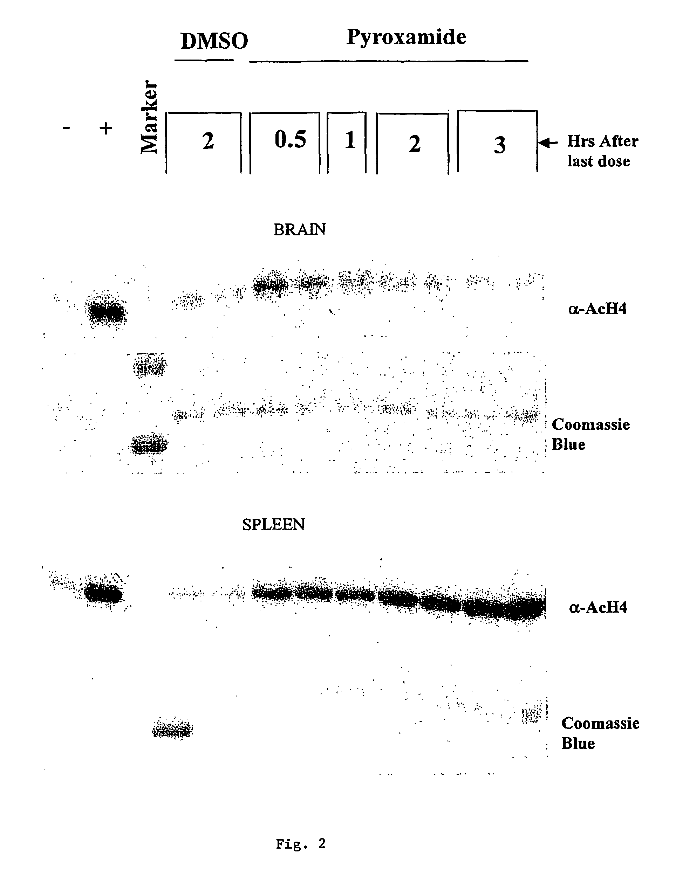

The foregoing objects, features and advantages of the invention will be apparent from the following more particular description of preferred embodiments of the invention, as illustrated in the accompanying drawings. The disclosure of Fig. 2 does not form part of the invention as claimed.

### DETAILED DESCRIPTION OF THE INVENTION

The present application is directed to inhibiting HDAC in the brain of a mammal. This comprises administering to an individual a HDAC inhibiting amount of a histone deacetylase inhibitor compound which is SAHA. The present application is further directed to treating diseases of the central nervous system (CNS) comprising administering to a individual in need of treatment a therapeutically effective amount of the inhibitor of HDAC. The disorder is cancer of the brain.

The individual is a mammal such as a primate or human.

Therapeutically effective amount as that term is used herein refers to an amount which elicits the desired therapeutic effect. The therapeutic effect is dependent upon the disease being treated and the results desired. As such, the therapeutic effect can be a decrease in the severity of symptoms associated with the disease and/or inhibition (partial or complete) of progression of the disease. Further, the therapeutic effect can be inhibition of HDAC in the brain. The amount needed to elicit the therapeutic response can be determined based on the age, health, size and sex of the patient. Optimal amounts can also be determined based on monitoring of the patient's response to treatment.

Generally, diseases of the central nervous system, are referred to as neurodegenerative, indicating that they are characterized by gradually evolving, relentlessly progressive neuronal death with many occurring for reasons that are still largely unknown. The identification of these diseases depends upon exclusion of such possible causative factors as infections, metabolic derangements, and intoxications. A considerable proportion of the disorders classed as neurogenerative are generic, with either dominant or recessive inheritance. Others, however, occur only sporadically as isolated instances in a given family. Classification of the degenerative diseases cannot be based upon any exact knowledge of cause or pathogenesis; their subdivision into individual syndromes rests on descriptive criteria based largely upon neuropathologic and clinical aspects.

However, research in the past decade has uncovered a new classification of inherited neurodegenerative diseases, the polyglutamine (polyQ) expansion diseases. In each, the underlying mutation is an expansion of a CAG trinucleotide repeat that encodes polyQ in the respective disease protein. All are progressive, ultimately fatal disorders that typically begin in adulthood and progress over 10 to 30 years. The clinical features and pattern of neuronal degeneration differ among the diseases, yet increasing evidence suggests that polyQ diseases share important pathogenic features. In particular, abnormal protein conformations promoted by polyQ expansion seem to be central to pathogenesis. This class of PolyQ expansion neurodegenerative disease are Huntington's Disease (HD), Dentatorubralpallidoluysian atrophy (DRPLA), spinal and bulbar muscular atrophy (SBMA), and five spinocerebellar ataxias (SCA1, SCA2, SCA3/MJD(Machado-Joseph Disease), SCA6 and SCA7). These diseases are listed in the general listing of neurodegenrative disease below. Many of these diseases not yet connected with PolyQ expansion are thought to result from abnormal protein folding and aggregation (*e.g.*, Alzheimer's disease).

Generally, neurodegenerative diseases can be grouped as follows:
I. Disorders characterized by progressive dementia in the absence of other prominent neurologic signs.
   A. Alzheimer's disease
   B. Senile dementia of the Alzheimer type
   C. Pick's disease (lobar atrophy)
II. Syndromes combining progressive dementia with other prominent neurologic abnormalities
   A. Mainly in adults
      1. Huntington's disease
      2. Multiple system atrophy combining dementia with ataxia and/or manifestations of Parkinson's disease
      3. Progressive supranuclear aplsy (Steel-Richardson-OlszewsKi)
      4. Diffuse Lewy body disease
      5. Corticodentatonigral degeneration
   B. Mainly in children or young adults
      1. Hallervorden-Spatz disease
      2. Progressive familial myoclonic epilepsy
III. Syndromes of gradually developing abnormalities of posture and movement
   A. Paralysis agitans (Parkinson's disease)
   B. Striatonigral degeneration
   C. Progressive supranuclear palsy
   D. Torsion dystonia (torsion spasm; dystonia musculorum deformans)
   E. Spasmodic torticollis and other dyskinesis
   F. Familial tremor
   G. Gilles de la Tourette syndrome
IV. Syndromes of progressive ataxia
   A. Cerebellar degenerations
      1. Cerebellar cortical degeneration
      2. Olivopontocerebellar atrophy (OPCA)
   B. Spinocerebellar degeneration (Friedreich's atazia and related disorders)
V. Syndrome of central autonomic nervous system failure (Shy-Drager syndrome)
VI. Syndromes of muscular weakness and wasting without sensory changes (motor neuron disease)
   A. Amyotrophic lateral sclerosis
   B. Spinal muscular atrophy
      1. Infantile spinal muscular atrophy (Werdnig-Hoffman)
      2. Juvenile spinal muscular atrophy (Wohlfart-Kugelberg-Welander)
      3. Other forms of familial spinal muscular atrophy
   C. Primary lateral sclerosis
   D. Hereditary spastic paraplegia
VII. Syndromes combining muscular weakness and wasting with sensory changes (progressive neural muscular atrophy; chronic familial polyneuropathies)
   A. Peroneal muscular atrophy (Charcot-Marie-Tooth)
   B. Hypertrophic interstitial polyneuropathy (Dejerine-Sottas)
   C. Miscellaneous forms of chronic progressive neuropathy
VIII. Syndromes of progressive visual loss
   A. Pigmentary degeneration of the retina (retinitis pigmentosa)
   B. Hereditary optic atrophy (Leber's disease)

As used herein, "brain cancer" refers to cancer resulting from both primary and metastatic brain tumors. Gliomas are primary brain tumors which arise from the glial cells in the brain and spinal cord, and are the most common primary brain tumors. Gliomas are classified into several groups based on the type of glial cell involved. For example, astrocytomas, which are the most common type of gliomas, are developed from astrocytes. Types of astrocytomas include well-differentiated, anaplastic, and glioblastoma multiforme. Other types of glioma include ependymomas, oligodendrogliomas, ganglioneuromas, mixed gliomas, brain stem gliomas, optic nerve gliomas, meningiomas, pineal tumors, pituitary adenomas, and primitive neuroectodermal tumors, such as medulloblastomas, neuroblastomas, pineoblastomas, medulloepitheliomas, ependymoblastomas and polar spongioblastomas. Non-glioma type tumors include chordomas and craniopharyngiomas.

Histone deacetylases (HDACs) as that term is used herein are enzymes which catalyze the removal of acetyl groups from lysine residues in the amino terminal tails of the nucleosomal core histones. As such, HDAC together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo*. HDAC can be divided into three classes based on structural homology. Class I HDACs (HDACs 1, 2, 3 and 8) bear similarity to the yeast RPD3 protein, are located in the nucleus and are found in complexes associated with transcriptional corepressors. Class II HDACs (HDACs 4, 5, 6, 7 and 9) are similar to the yeast HEA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Histone deacetylase inhibitors or HDAC inhibitors, as that term is used herein are compounds which are capable of inhibiting the deacetylation of histones *in vivo*, *in vitro* or both. As such, HDAC inhibitors inhibit the activity of at least one histone deacetylase. As a result of inhibiting the deacetylation of at least one histone, an increase in acetylated histone occurs and accumulation of acetylated histone is a suitable biological marker for assessing the activity of HDAC inhibitors. Therefore, procedures which can assay for the accumulation of acetylated histones can be used to determine the HDAC inhibitory activity of compounds of interest. It is understood that compounds which can inhibit histone deacetylase activity can also bind to other substrates and as such can inhibit other biologically active molecules such as enzymes. It is also understood, that the HDAC inhibitor SAHA, suitable for use in the invention described herein, is capable of crossing the Blood Brain Barrier.

For example, in patients receiving HDAC inhibitors, the accumulation of acetylated histones in peripheral mononuclear cells as well as in tissue treated with HDAC inhibitors can be determined against a suitable control.

HDAC inhibitory activity of a particular compound can be determined *in vitro* using, for example, an enzymatic assays which shows inhibition of at least one histone deacetylase. Further, determination of the accumulation of acetylated histones in cells treated with a particular composition can be determinative of the HDAC inhibitory activity of a compound.

Assays for the accumulation of acetylated histones are well known in the literature. See, for example, Marks, P.A. et al., J. Natl. Cancer Inst., 92:1210-1215, 2000, Butler, L.M. et al., Cancer Res. 60:5165-5170 (2000), Richon, V. M. et al., Proc. Natl. Acad. Sci., USA, 95:3003-3007, 1998, and Yoshida, M. et al., J. Biol. Chem., 265:17174-17179, 1990.

For example, an enzymatic assay to determine the activity of a histone deacetylase inhibitor compound can be conducted as follows. Briefly, the effect of an HDAC inhibitor compound on affinity purified human epitope-tagged (Flag) HDAC1 can be assayed by incubating the enzyme preparation in the absence of substrate on ice for about 20 minutes with the indicated amount of inhibitor compound. Substrate ([³H]acetyl-labelled murine erythroleukemia cell-derived histone) can be added and the sample can be incubated for 20 minutes at 37°C in a total volume of 30 µL. The reaction can then be stopped and released acetate can be extracted and the amount of radioactivity release determined by scintillation counting. An alternative assay useful for determining the activity of a histone deacetylase inhibitor compound is the "HDAC Fluorescent Activity Assay;Drug Discovery Kit-AK-500" available from BIOMOL® Research Laboratories, Inc., Plymouth Meeting, PA.

In vivo studies can be conducted as follows. Animals, for example mice, can be injected intraperitoneally with an HDAC inhibitor compound. Selected tissues, for example brain, spleen, liver etc..., can be isolated at predetermined times, post administration. Histones can be isolated from tissues essentially as described by Yoshida et al., J. Biol. Chem. 265:17174-17179, 1990. Equal amounts of histones (about 1 µg) can be electrophoresed on 15% SDS-polyacrylamide gels and can be transferred to Hybond-P filters (available from Amersham). Filters can be blocked with 3% milk and can be probed with a rabbit purified polyclonal anti-acetylated histone H4 antibody (αAc-H4) and anti-acetylated histone H3 antibody (αAc-H3) (Upstate Biotechnology, Inc.). Levels of acetylated histone can be visualized using a horseradish peroxidase-conjugated goat anti-rabbit antibody (1:5000) and the SuperSignal chemiluminescent substrate (Pierce). As a loading control for the histone protein, parallel gels can be run and stained with Coomassie Blue (CB).

In addition, hydroxamic acid-based HDAC inhibitors have been shown to up regulate the expression of the p21^{WAF1} gene. The p21^{WAF1} protein is induced within 2 hours of culture with HDAC inhibitors in a variety of transformed cells using standard methods. The induction of the p21^{WAF1} gene is associated with accumulation of acetylated histones in the chromatin region of this gene. Induction of p21^{WAF1} can therefore be recognized as involved in the G1 cell cycle arrest caused by HDAC inhibitors in transformed cells.

Typically, HDAC inhibitors fall into four general classes: 1) short-chain fatty acids (*e.g.*, 4-phenylbutyrate and valproic acid); hydroxamic acids (*e.g.*, SAHA, Pyroxamide, trichostarin A (TSA), oxamflatin, Scriptaid and CHAPs, such as, CHAP1 and CHAP 31); 3) cyclic tetrapeptides (Trapoxin A and Apicidin; 4) benzamides (*e.g.*, MS-275). Examples of such compounds can be found in U.S. Patent Nos. 5,369,108, issued on November 29, 1994,5,700,811, issued on December 23, 1997, 5,773,474, issued on June 30, 1998 and 5,932,616 issued onAugust 3, 1999 all to Breslow *et al.*, U.S. Patent Nos. 5,055,608, issued on October 8, 1991, 5, 175, 191, issued on December 29,1992 and 5,608,108 issued on March 4,1997 all to Marks *et al.*, as well as, Yoshida, M., et al., Bioassays 17, 423-430 (1995), Saito, A., et al., PNAS USA 96,4592-4597, (1999), Furamai R. et al., PNAS USA 98 (1), 87-92 (2001), Komatsu, Y., et al., Cancer Res. 61(11), 4459-4466 (2001), Su, G.H., et al., Cancer Res. 60, 3137-3142 (2000), Lee, B.I. et al., Cancer Res. 61(3), 931-934, Suzuki, T., et al., J. Med. Chem. 42(15), 3001-3003 (1999), published PCT Application WO 01/18171 published on March 15, 2001 to Sloan-Kettering Institute for Cancer Research and The Trustees of Columbia University, published PCT Applications WO 01/38322 (published May 31, 2001), WO 01/70675 (published on September 27,2001) and WO 00/71703 (published on November 30, 2000) all to Methylgene, Inc., published PCT Application WO 00/21979 published on October 8, 1999 to Fujisawa Pharmaceutical Co., Ltd. an published PCT Application WO 98/40080 published on March 11, 1998 to Beacon Laboratories, L.L.C. It is understood that the HDAC inhibiton SAHA, suitable for use in the invention described herein, must be capable of permeabilizing the blood brain barrier such that therapeutic levels of inhibitor can enter the CNS.

The hydroxamic acid based HDAC inhibitor is suberoylanilide hydroxamic acid (SAHA) .SAHA has been shown to bind directly in the catalytic pocket of the histone deacetylase enzyme. SAHA induces cell cycle arrest, differentiation and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carcinogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

SAHA can be represented by the following structural formula:

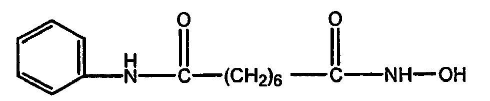

Other HDAC inhibitors are pyroxamide, that can be represented by the following structural formula: 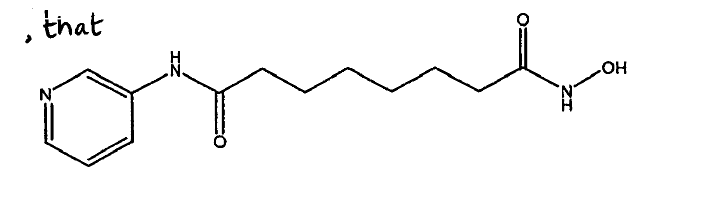 and CBHA, that can be represented by the structural formula:

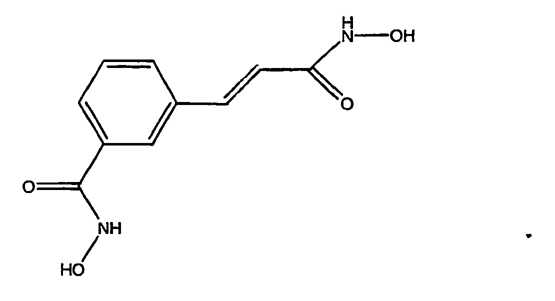

Another HDAC inhibitor can be represented by Formula I: 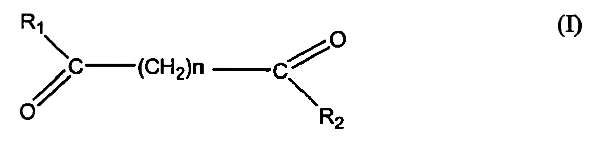 wherein R₁ and R₂ can be the same or different; when R₁ and R₂ are the same, each is a substituted or unsubstituted arylamino, cycloalkylamino, pyridineamino, piperidino, 9-purine-6-amine or thiazoleamino group; when R₁ and R₂ are different R₁=R₃-N-R₄, wherein each of R₃ and R₄ are independently the same as or different from each other and are a hydrogen atom, a hydroxyl group, a substituted or unsubstituted, branched or unbranched alkyl, alkenyl, cycloalkyl, aryl alkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8.

Other HDAC inhibitors can be represented by formula II: 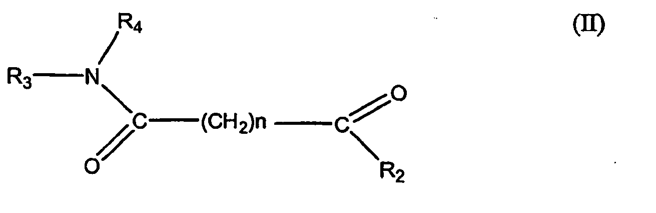 wherein each of R₃ and R₄ are independently the same as or different from each other and are a hydrogen atom, a hydroxyl group, a substituted or unsubstituted, branched or unbranched alkyl, alkenyl, cycloalkyl, arylalkyloxy, aryloxy, arylalkyloxy or pyridine group, or R₃ and R₄ bonded together to form a piperidine group, R₂ is a hydroxylamino, hydroxyl, amino, alkylamino, dialkylamino or alkyloxy group and n is an integer from about 4 to about 8.

In a particular embodiment of Formula II, R₂ is a hydroxylamino, hydroxyl, amino, methylamino, dimethylamino or methyloxy group and n is 6. In yet another embodiment of Formula II, R₄ is a hydrogen atom, R₃ is a substituted or unsubstituted phenyl and n is 6. In further embodiments of Formula II, R₄ is hydrogen and R₃ is an α, β-, or γ-pyridine.

In other specific embodiments of Formula II, R₄ is a hydrogen atom and R₃ is a cyclohexyl group; R₄ is a hydrogen atom and R₃ is a methoxy group; R₃ and R₄ each bond together to form a piperidine group; R₄ is a hydrogen atom and R₃ is a hydroxyl group; R₃ and R₄ are both a methyl group and R₃ is phenyl and R₄ is methyl.

Further HDAC inhibitors can be represented by structural Formula III: 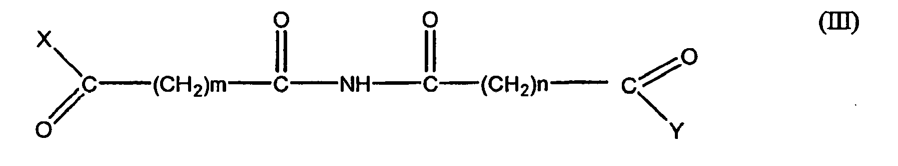 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino, or aryloxyalkylamino group; R is a hydrogen atom, a hydroxyl, group, a substituted or unsubstituted alkyl, arylalkyloxy, or aryloxy group; and each ofm and n are independently the same as or different from each other and are each an integer from about 0 to about 8.

In a particular embodiment, which does not form part of the invention as claimed the HDAC inhibitor is a compound of Formula III wherein X, Y and R are each hydroxyl and both m and n are 5.

Yet other HDAC inhibitor compounds can be represented by structural Formula IV: 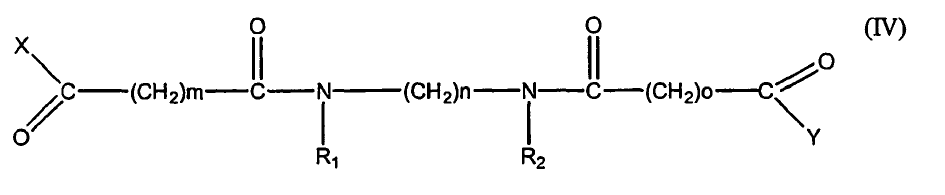 wherein each of X and Y are independently the same as or different from each other and are a hydroxyl, amino or hydroxylamino group, a substituted or unsbustituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m, n and o are independently the same as or different from each other and are each an integer from about 0 to about 8.

Other HDAC inhibitors include compounds having structural Formula V: 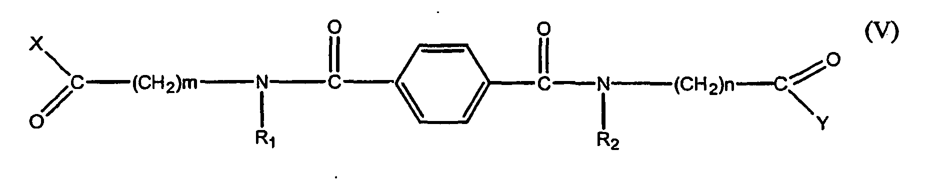 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; each of R₁ and R₂ are independently the same as or different from each other and are a hydrogen atom, a hydroxyl group, a substituted or unsubstituted alkyl, aryl, alkyloxy, or aryloxy group; and each of m and n are independently the same as or different from each other and are each an integer from about 0 to about 8.

Further HDAC inhibitors can have structural Formula VI: 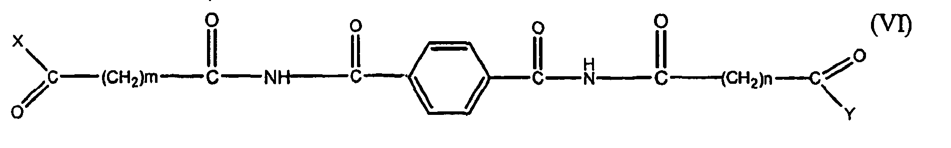 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; and each of m and n are independently the same as or different from each other and are each an integer from about 0 to about 8.

HDAC inhibitors can have structural Formula VII: 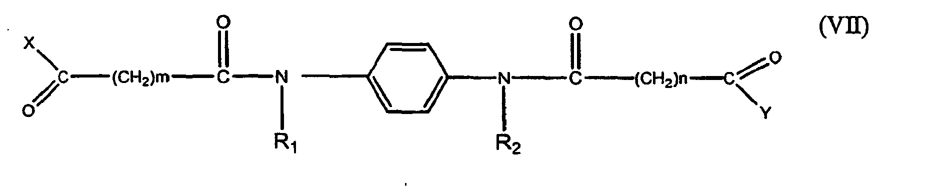 wherein each of X and Y are independently the same as or different from each other and are a hydroxyl, amino or hydroxylamino group, a substituted or unsubstituted alkyloxy, alkylamino, dialkylamino, arylamino, alkylarylamino, alkyloxyamino, aryloxyamino, alkyloxyalkylamino or aryloxyalkylamino group; R₁ and R₂ are independently the same as or different from each other and are a hydrogen atom, a hydroxyl group, a substituted or unsubstituted alkyl, arylalkyloxy or aryloxy group; and each of m and n are independently the same as or different from each other and are each an integer from about 0 to about 8.

HDAC inhibitors can have structural Formula VIII: 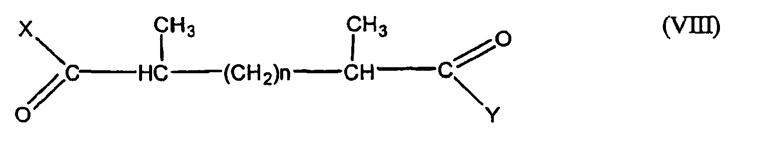 wherein each of X an Y are independently the same as or different from each other and are a hydroxyl, amino or hydroxylamino group, a substituted or unsubstituted alkyloxy, alkylamino, dialkylamino, arylamino, alkylarylamino, or aryloxyalkylamino group; and n is an integer from about 0 to about 8.

Additional compounds include those represented by Formula IX: 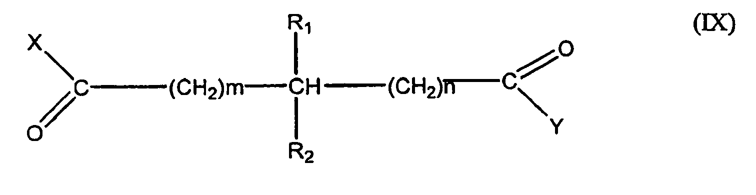 wherein Each of X and Y are independently the same as or different from each other and are a hydroxyl, amino or hydroxylamino group, a substituted or unsbustituted alkyloxy, alkylamino, dialkylamino, arylamino, alkylarylamino, alkyloxyamino, aryloxyamino, alkyloxyalkyamino or aryloxyalkylamino group; each of R₁ and R₂ are independently the same as or different from each other and are a hydrogen atom, a hydroxyl group, a substitituted or unsubstituted alkyl, aryl, alkyloxy, aryloxy, carbonylhydroxylamino or fluoro group; and each ofm and n are independently the same as or different from each other and are each an integer from about 0 to about 8.

Further HDAC inhibitors include compounds having structural Formula X: 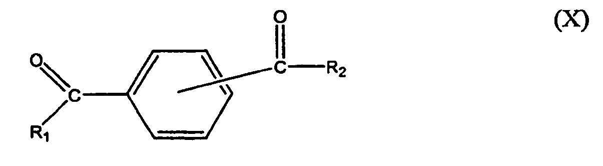 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group. In a particular embodiment, not part of the invention as claimed, the HDAC inhibitor is a compound of structural Formula X wherein R₁ and R₂ are both hydroxylamino.

Another HDAC inhibitor has structural Formula XI: 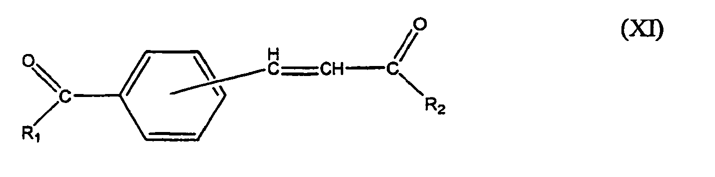 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group.

This HDAC inhibitor can be compound of structural Formula XI wherein R₁ and R₂ are both hydroxylamino.

Further HDAC inhibitors include compounds represented by structural Formula XII: 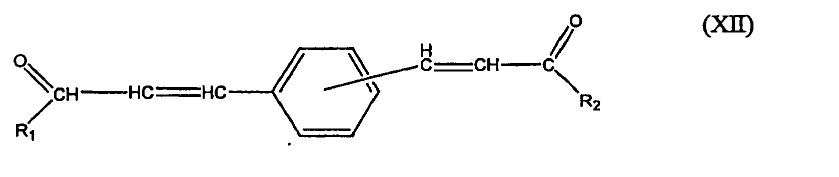 wherein each of R₁ and R₂ are independently the same as or different from each other and are a hydroxyl, alkyloxy, amino, hydroxylamino, alkylamino, dialkylamino, arylamino, alkylarylamino, alkyloxyamino, aryloxyamino, alkyloxyalkylamino, or aryloxyalkylamino group.

This HDAC inhibitor can be compound of structural Formula XII wherein R₁ and R₂ are both hydroxylamino.

Additional compounds include those represented by structural Formula XIII: 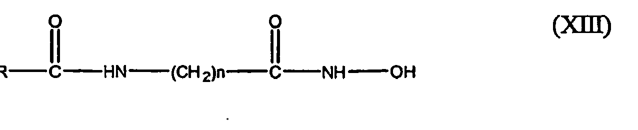 wherein R is a substituted or unsbustituted phenyl, piperidine, thiazole, 2-pyridine, 3-pyridine or 4-pyridine and n is an integer from about 4 to about 8.

The HDAC inhibitors can be represented by structural Formula (XIV): 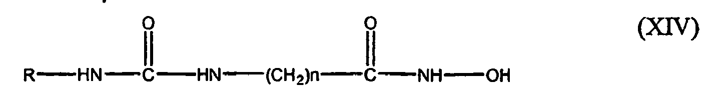 wherein R is a substituted or unsubstituted phenyl, pyridine, piperidine or thiazole group and n is an integer from about 4 to aobut 8 or a pharmaceutically acceptable salt thereof. In a particular embodiment, R is phenyl and n is 5. In another embodiment, n is 5 and R is 3-chlorophenyl.

In structural formulas I-XIV, substituted phenyl, refers to a phenyl group which can be substituted with, for example, but not limited to a methyl, cyano, nitro, trifluoromethyl, amino, aminocarbonyl, methylcyano, halogen, e.g., chloro, fluoro, bromo, iodo, 2,3-difluoro, 2,4-difluoro, 2,5-difluoro, 3,4-difluoro, 3,5-difluoro, 2,6-difluoro, 1,2,3-trifluoro, 2,3,6-trifluoro, 2,3,4,5,6-pentafluoro, azido, hexyl, t-butyl, phenyl, carboxyl, hydroxyl, methyloxy, benzyloxy, phenyloxy, phenylaminooxy, phenylaminocarbonyl, methyloxycarbonyl, methylaminocarbonyl, dimethylamino, dimethylaminocarbonyl or hydroxyaminocarbonyl group.

Other HDAC inhibitors can be represented by structural Formula XV: 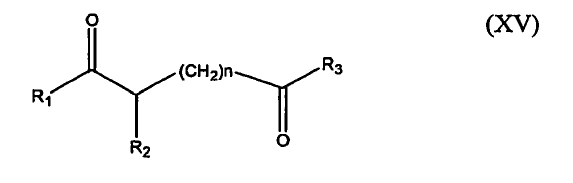 wherein each of R₁ and R₂ is directly attached or through a linker and is substituted or unsubstituted, aryl (e.g. naphthyl, phenyl), cycloalkyl, cycloalkylamino, pyridineamino, piperidino, 9-purine-6-amine, thiazoleamino group, hydroxyl, branched or unbranched alkyl, alkenyl, alkyloxy, aryloxy, arylalkyloxy, or pyridine group; n is an integer from about 3 to about 10 and R₃ is a hydroxamic acid, hydroxylamino, hydroxyl, amino, alkylamino or alkyloxy group.

The linker can be an amide moiety, -O-, -S-, -NH- or -CH₂-.

In certain embodiments, R₁ is -NH-R₄ wherein R₄ is substituted or unsubstituted, aryl (e.g., naphthyl, phenyl), cycloalkyl, cycloalkylamino, pyridineamino, piperidino, 9-purine-6-amine, thiazoleamino group, hydroxyl, branched or unbranched alkyl, alkenyl, alkyloxy, aryloxy, arylalkyloxy or pyridine group.

Further and more specific HDAC inhibitors of Formula XV, include those which can be represented by Formula XVI: 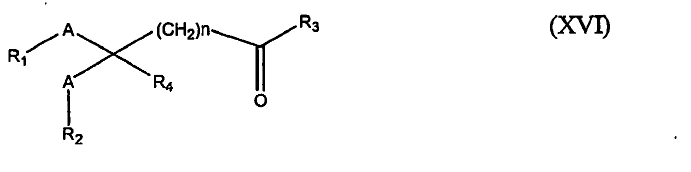 wherein each of R₁ and R₂ is, substituted or unsubstituted, aryl (e.g., phenyl, naphthyl), cycloalkyl, cycloalkylamino, pyridneamino, piperidino, 9-purine-6-amine, thiasoleamino group, hydroxyl, branched or unbranched alkyl, alkenyl, alkyloxy, aryloxy, arylalkyloxy or pyridine group; R₃ is hydroxamic acid, hydroxylamino, hydroxyl, amino, alkylamino or alkyloxy group; R₄ is hydrogen, halogen, phenyl or a cycloalkyl moiety; and A can be the same or different and represents an amide moiety, -O-, -S-, -NR₅- or -CH₂-where R₅ is a substitute or unsubstituted C₁-C₅ alkyl and n is an integer from 3 to 10.

For example, further compounds having a more specific structure within Formula XVI can be represented by structural Formula XVII: 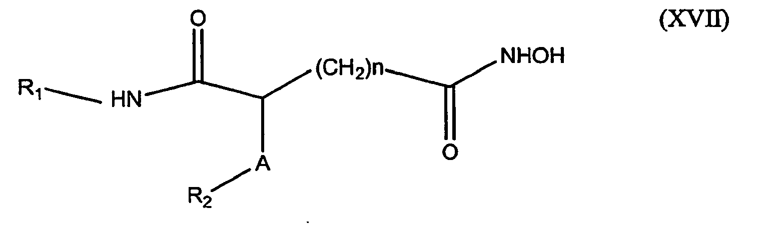 wherein A is an amide moiety, R₁ and R₂ are each selected from substituted or unsubstituted aryl (e.g., pheny, naphthyl), pyridineamino, 9-purine-6-amine, thiazoleamino, aryloxy, arylalkyloxy, or pyridine and n is an integer from 3 to 10. For example, the compound can have the formula 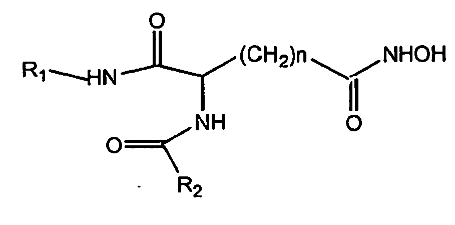 or

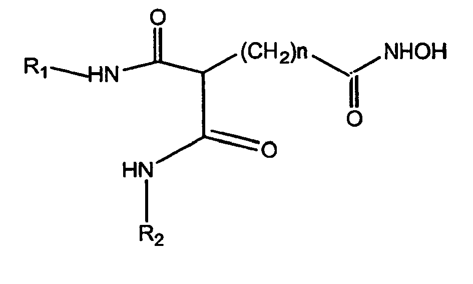

Another the HDAC inhibitor can have the Formula XVIII: 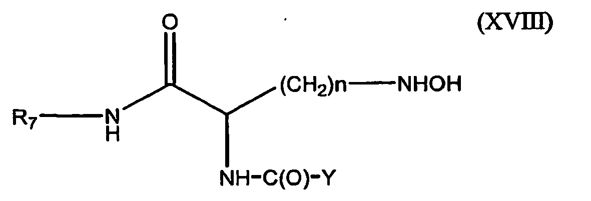 wherein R₇ is selected from substituted or unsubstituted aryl (e.g., phenyl or naphthyl), pyridineamino, 9-purine-6-amine, thiazoleamino, aryloxy, arylalkyloxy or pyridine and n is an integer from 3 to 10 and Y is selected from 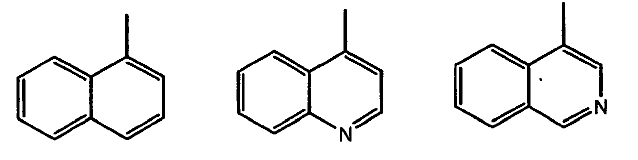 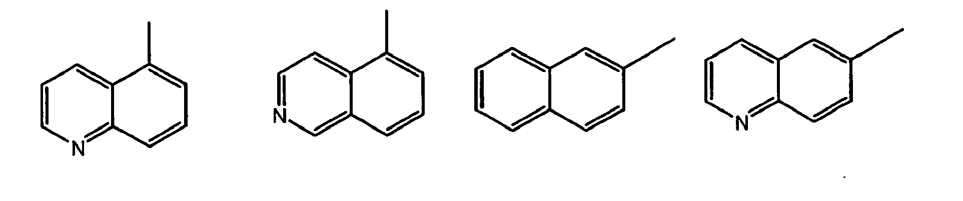 or a pharmaceutically acceptable salt thereof.

The HDAC inhibitor compound can have Formula XIX: 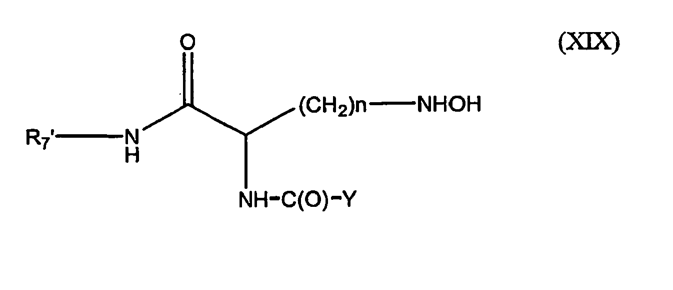 wherein n is an integer from 3 to 10, Y is selected from 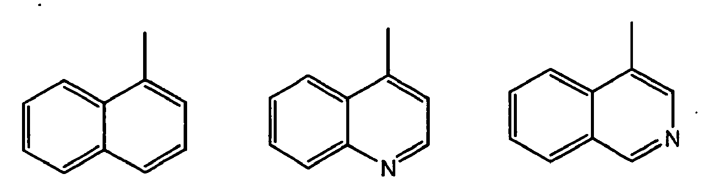 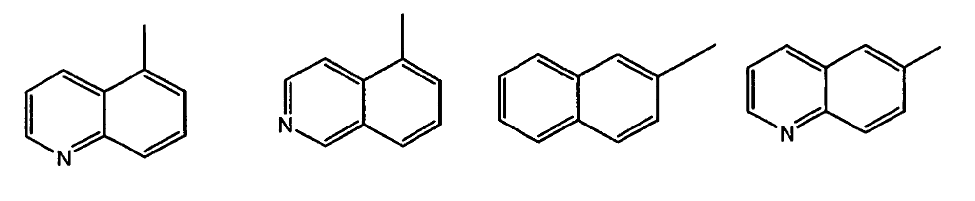 and R₇' is selected from 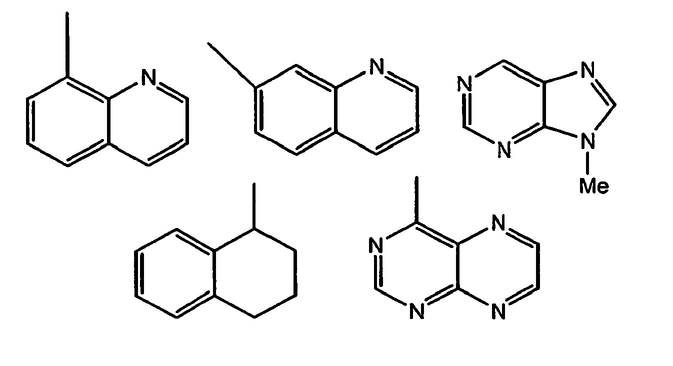 or a pharmaceutically acceptable salt thereof.

Further compounds can be represented by structural Formula XX: 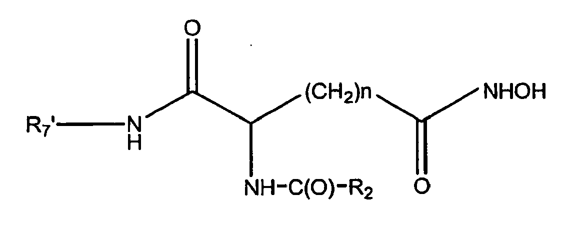 wherein R₂ is selected from substituted or unsubstituted aryl, substituted or unsubstituted naphtha, pyridineamino, 9-purine-6-amine, thiazoleamino, substituted or unsubstituted aryloxy, substituted or unsubstituted arylalkyloxy or pyridine and n is an integer from 3 to 10 and R₇' is selected from 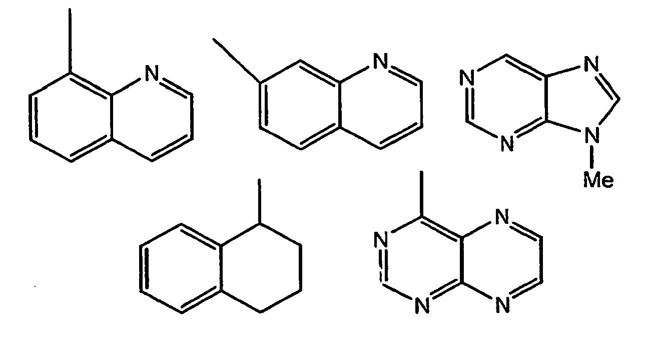 or a pharmaceutically acceptable salt thereof.

Further HDAC inhibitors can be represented by structural Formula XXI: 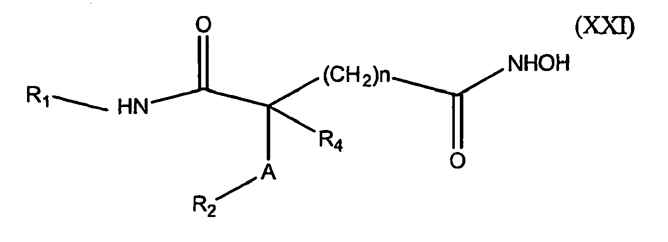 wherein A is an amide moiety, R₁ and R₂ are each selected from substituted or unsubstituted aryl, naphtha, pyridineamino, 9-purine-6-amine, thiazoleamino, aryloxy, arylalkyloxy or pyridine, R₄ is hydrogen, a halogen, a phenyl or a cycloalkyl moiety and n is an integer from 3 to 10 or a pharmaceutically acceptable salt thereof.

For example, a compound of Formula XXI can be represented by the structure: 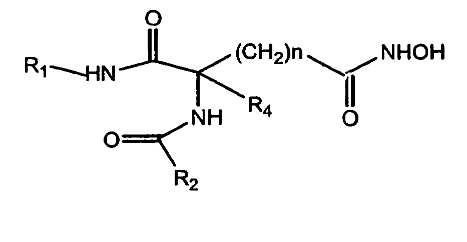 or can be represented by the structure: 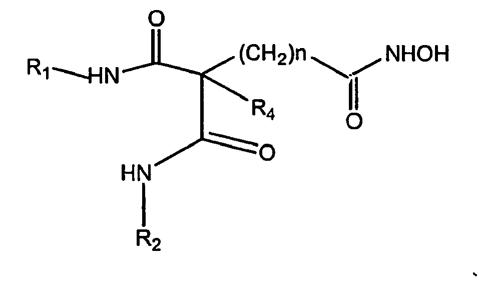 wherein R₁, R₂, R₄ and n have the meanings of Formula XXI.

Further, HDAC inhibitors having the structural Formula XXII: 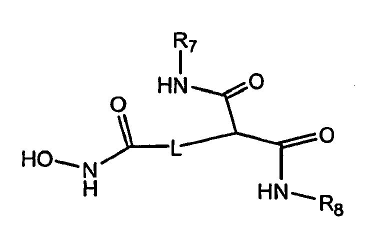 wherein L is a linker selected from the group consisting of -(CH2)ₙ-, -(CH=CH)ₘ-, - phenyl-, -cycloalkyl-, or any combination thereof; and wherein each of R₇ and R₈ are independently substituted or unsubstituted, aryl, naphtha, pyridineamino, 9-purine-6-amine, thiazoleamino group, aryloxy, arylalkyloxy, or pyridine group, n is an integer from 3 to 10 and m is an integer from 0-10. For example, a compound of Formula XXII can be:

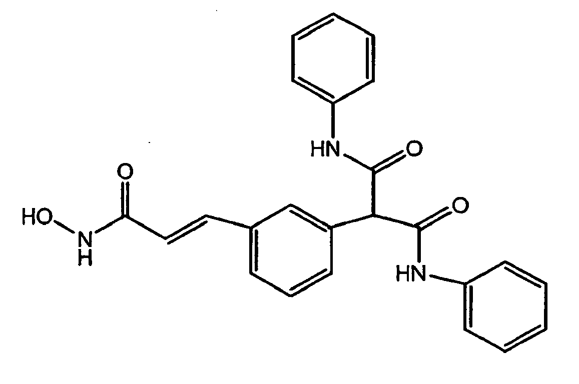

Other HDAC inhibitors include those shown in the following more specific formulas: 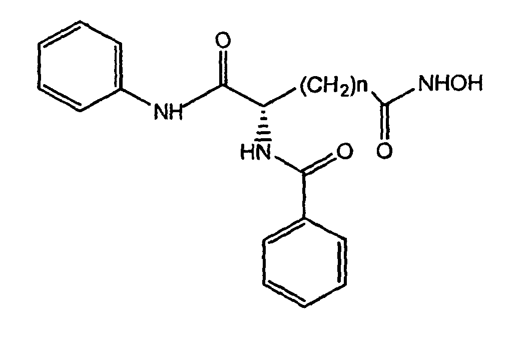 wherein n is an integer from 3 to 10; 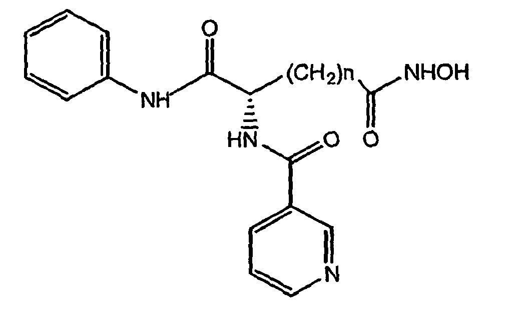 wherein n is an integer from 3 to 10; 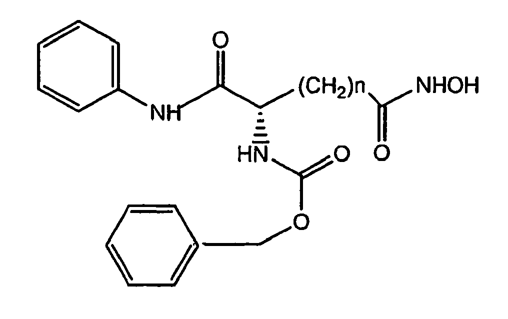 wherein n is an integer from 3 to 10; 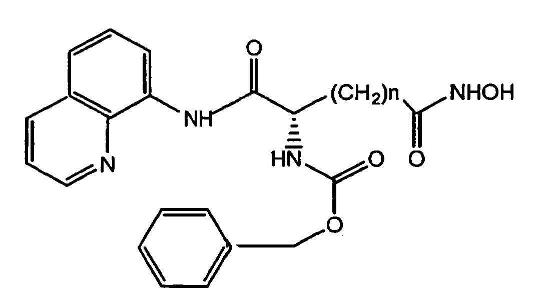 wherein n is an integer from 3 to 10; 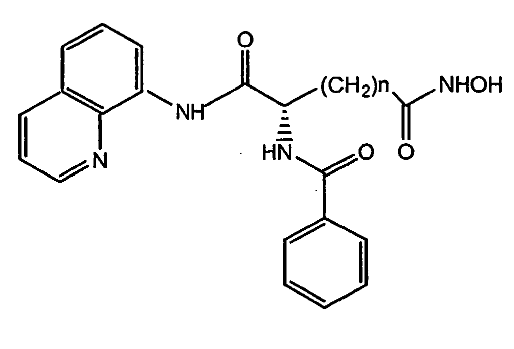 wherein n is an integer from 3 to 10; or a pharmaceutically acceptable salt thereof.

Further specific HDAC inhibitors include 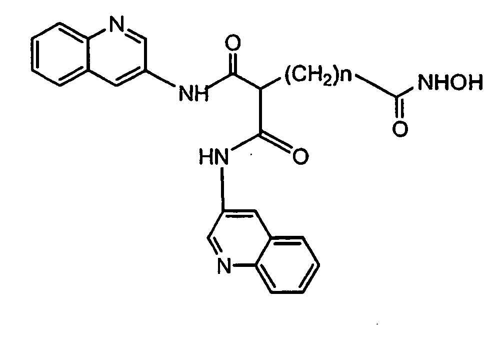 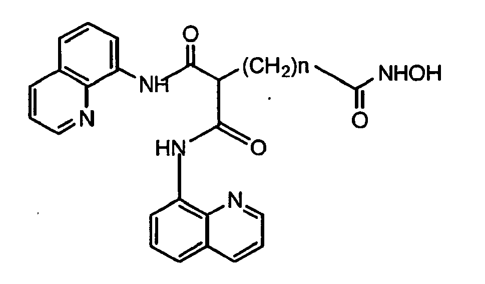 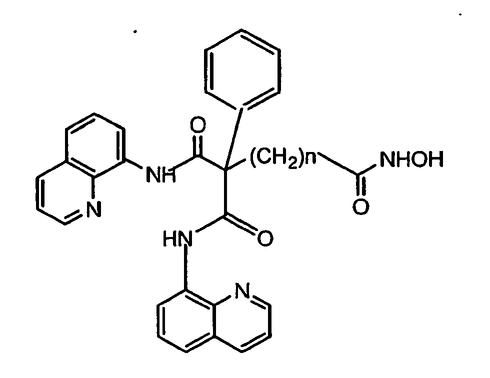 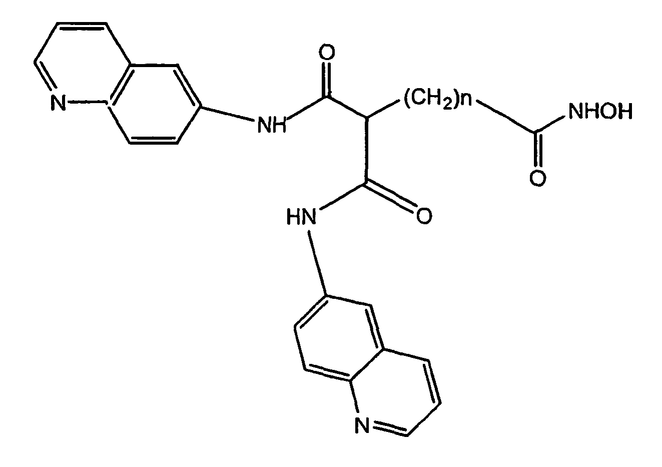 wherein n in each is an integer from 3 to 10 and the compound 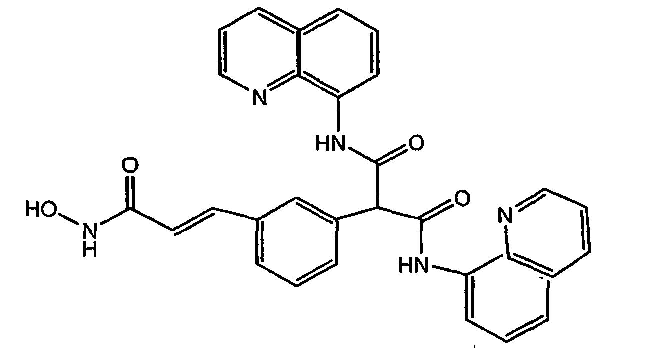 and pharmaceutically acceptable salts thereof.

HDAC inhibitors can have the following specific structures : 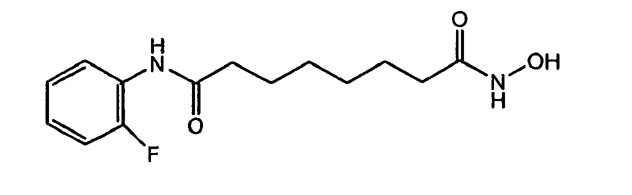 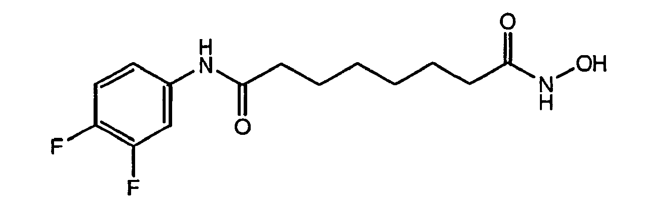 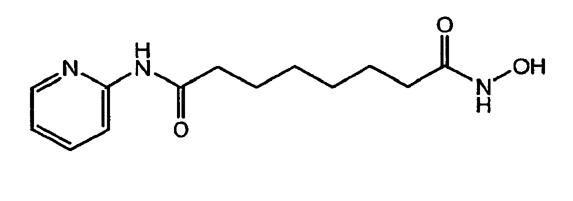 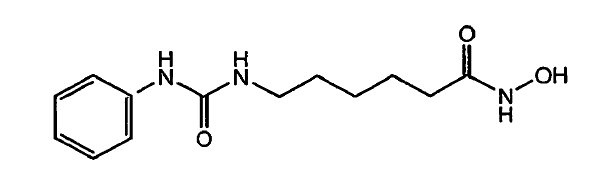 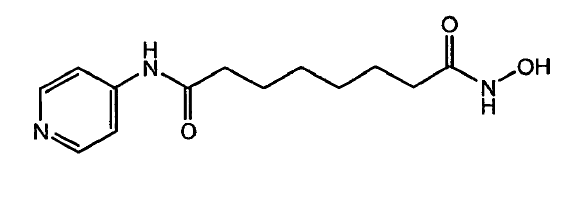 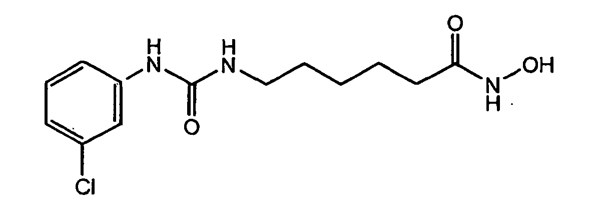 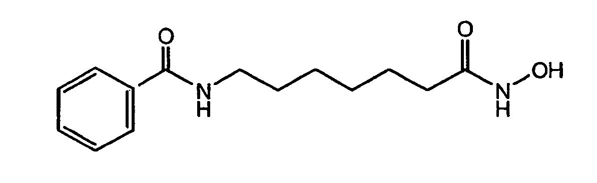 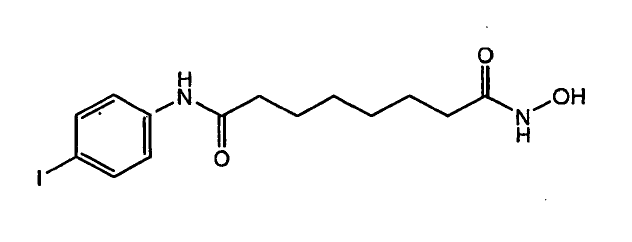 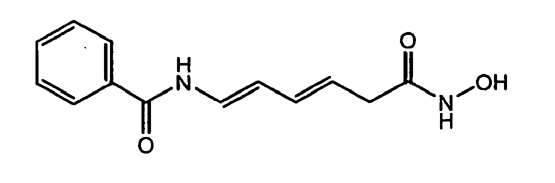 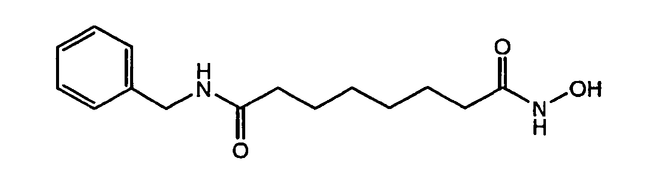 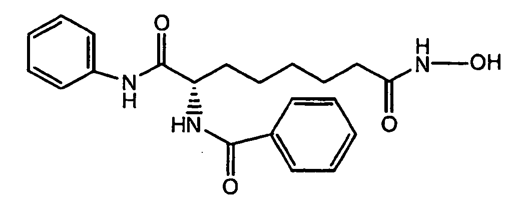 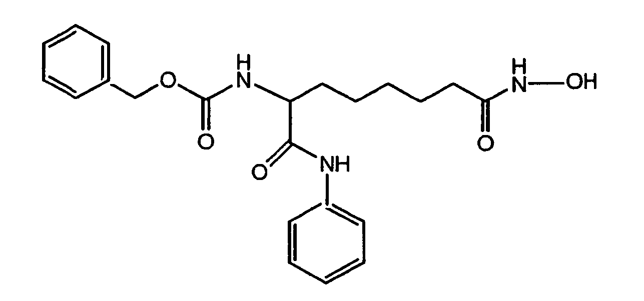 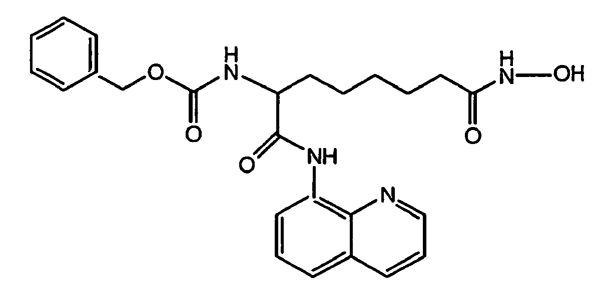 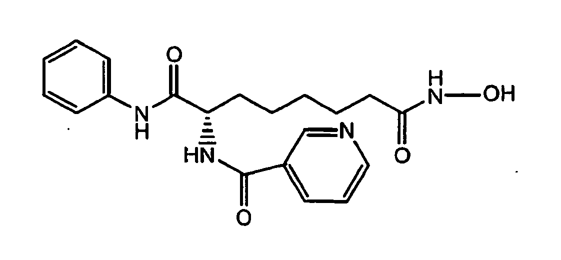 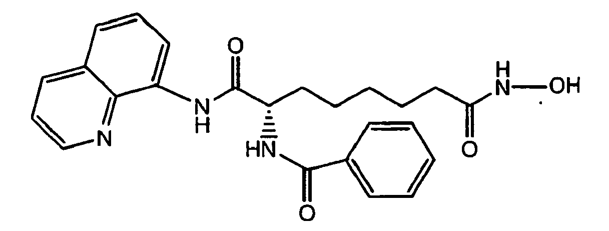 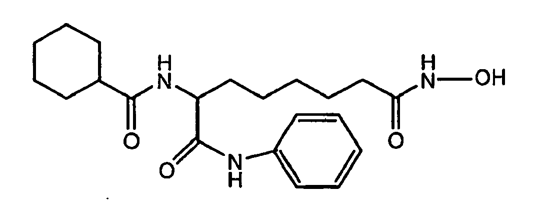 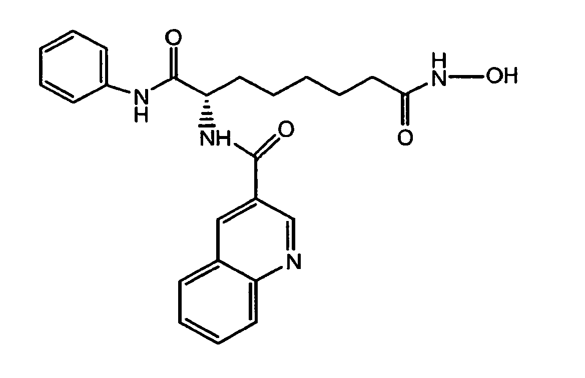 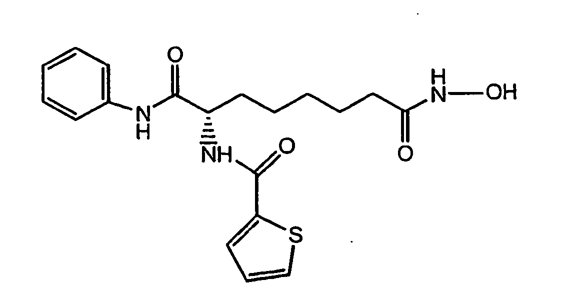 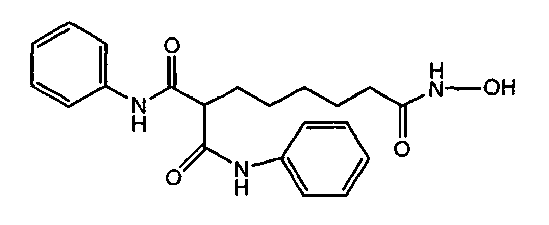 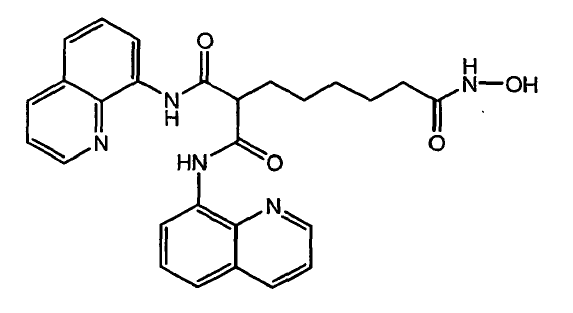 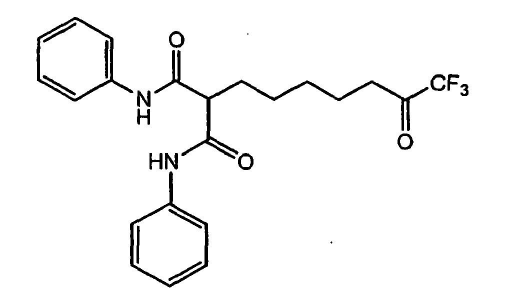 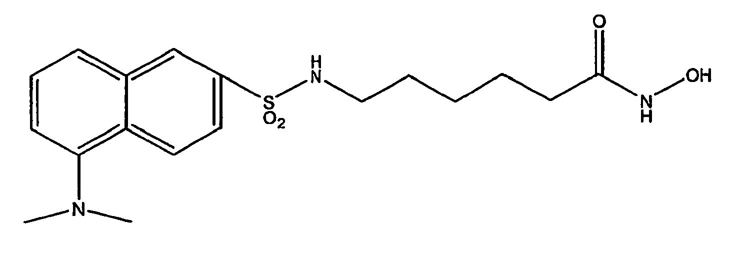 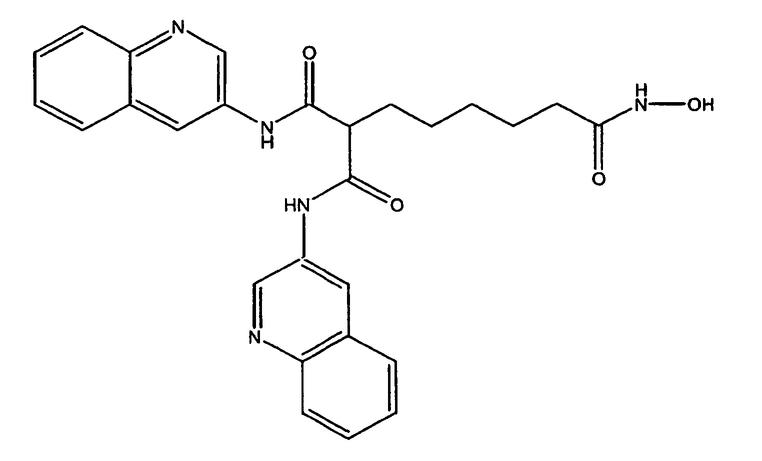 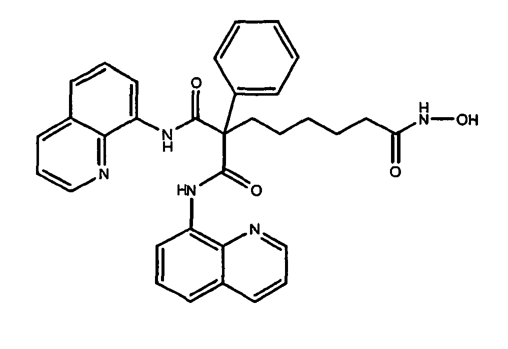 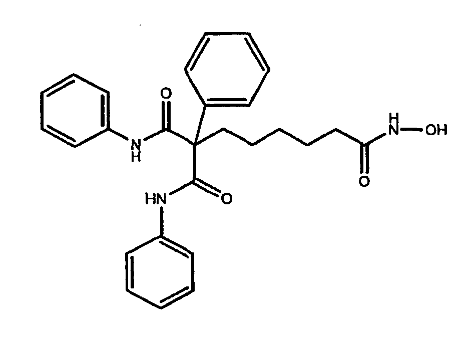 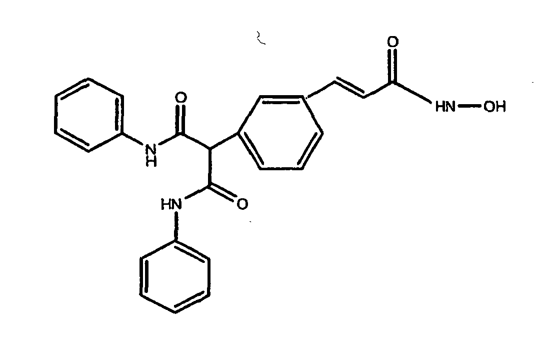 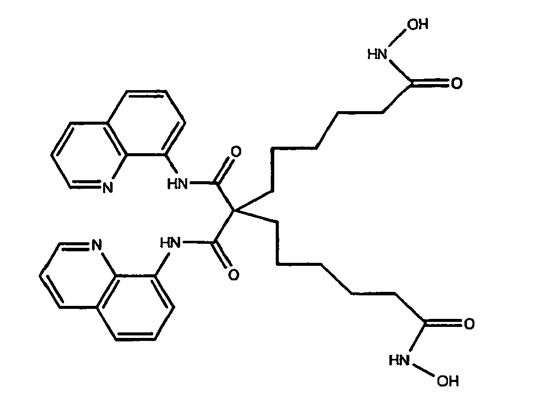 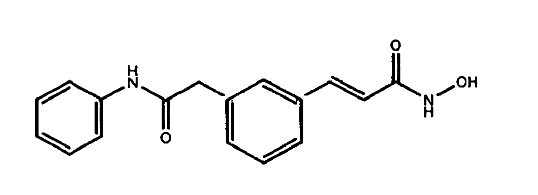 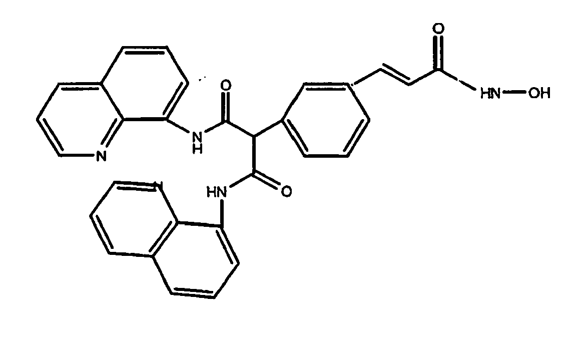 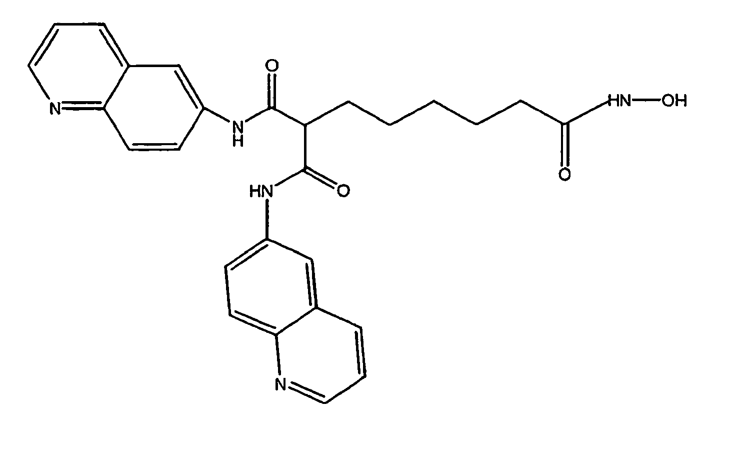 and pharmaceutically acceptable salts thereof.

The HDAC inhibitor of the invention is to be administered in such oral forms as tablets, capsules (each of which includes sustained release or timed release formulations), pills, powders, granules, elixers, tinctures, suspensions, syrups, and emulsions.

The HDAC inhibitor can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The HDAC inhibitor can also be delivered by the use of monoclonal antibodies as individual carriers to which the compound molecules are coupled.

The HDAC inhibitor can also be prepared with soluble polymers as targetable drug carriers. Such polymers can include polyvinlypyrrolidone, pyran copolymer, polyhydroxy-propyl-methacrylamide-phenol, polyhydroxyethyl-aspartamide-phenol, or polyethyleneoxide-polylysine substituted with palmitoyl residues. Furthermore, the HDAC inhibitor can be prepared with biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The dosage regimen utilizing the HDAC inhibitor can be selected in accordance with a variety of factors including type, species, age, weight, sex and the disorder being treated; the severity of the condition to be treated; the route of administration; the renal and hepatic function of the patient; and the particular compound or salt thereof employed. An ordinarily skilled physician or veterinarian can readily determine and prescribe the effective amount of the drug required to treat, for example, to prevent, inhibit (fully or partially) or arrest the progress of the disorder.

Oral dosages of the HDAC inhibitor, when used to treat the neurodegenerative diseases described herein or brain cancer, can range between about 2 mg to about 2000 mg per day, such as from about 20 mg to about 2000 mg per day, such as from about 200 mg to about 2000 mg per day. For example, oral dosages can be about 2, about 20, about 200, about 400, about 800, about 1200, about 1600 or about 2000 mg per day. It is understood that the total amount per day can be administered in a single dose or can be administered in multiple dosings such as twice, three or four times per day.

For example, a patient can receive between about 2 mg/day to about 2000 mg/day, for example, from about 20-2000 mg/day, such as from about 200 to about 2000 mg/day, for example from about 400 mg/day to about 1200 mg/day. A suitably prepared medicament for once a day administration can thus contain between about 2 mg and about 2000 mg, such as from about 20 mg to about 2000 mg, such as from about 200 mg to about 1200 mg, such as from about 400 mg/day to about 1200 mg/day. The HDAC inhibitor can be administered in a single dose or in divided doses of two, three, or four times daily. For administration twice a day, a suitably prepared medicament would therefore contain half of the needed daily dose.

The HDAC inhibitor can be administered as active ingredients in admixture with suitable pharmaceutical diluents, excipients or carriers (collectively referred to herein as "carrier" materials) suitably selected with respect to the intended form of administration, that is, oral tablets, capsules, elixers, syrups and the like, and consistent with conventional pharmaceutical practices.

For instance, for oral administration in the form of a tablet or capsule, the HDAC inhibitor can be combined with an oral, non-toxic, pharmaceutically acceptable, inert carrier such as lactose, starch, sucrose, glucose, methyl cellulose, microcrystalline cellulose, sodium croscarmellose, magnesium stearate, dicalcium phosphate, calcium sulfate, mannitol, sorbitol and the like or a combination thereof; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn-sweeteners, natural and synthetic gums such as acacia, tragacanth or sodium alginate, carboxymethylcellulose, microcrystalline cellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch methyl cellulose, agar, bentonite, xanthan gum and the like.

Suitable pharmaceutically acceptable salts of the histone deacetylase compound described herein and suitable for use in the method of the invention, i.e. SAHA, are conventional non-toxic salts and can include a salt with a base or an acid addition salt such as a salt with an inorganic base, for example, an alkali metal salt (e.g. lithium salt, sodium salt, potassium salt, etc.), an alkaline earth metal salt (e.g. calcium salt, magnesium salt, etc.), an ammonium salt; a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.) etc.; an inorganic acid addition salt (e.g. hydrochloride, hydrobromide, sulfate, phosphate, etc.); an organic carboxylic or sulfonic acid addition salt (e.g. formate, acetate, trifluoroacetate, maleate, tartrate, methanesulfonate, benzenesulfonate, p-toluenesulfonate, etc.); a salt with a basic or acidic amino acid (e.g. arginine, aspartic acid, glutamic acid, etc.) and the like.

It is understood that standard therapy agents for the disease being treated, can be administered in combination with the HDAC inhibitor suitable for use in the invention. These standard agents can be administered by the methods described above for the HDAC inhibitor. The combination of agents, however, can be administered using the same or different methods.

### EXPERIMENTAL METHODS

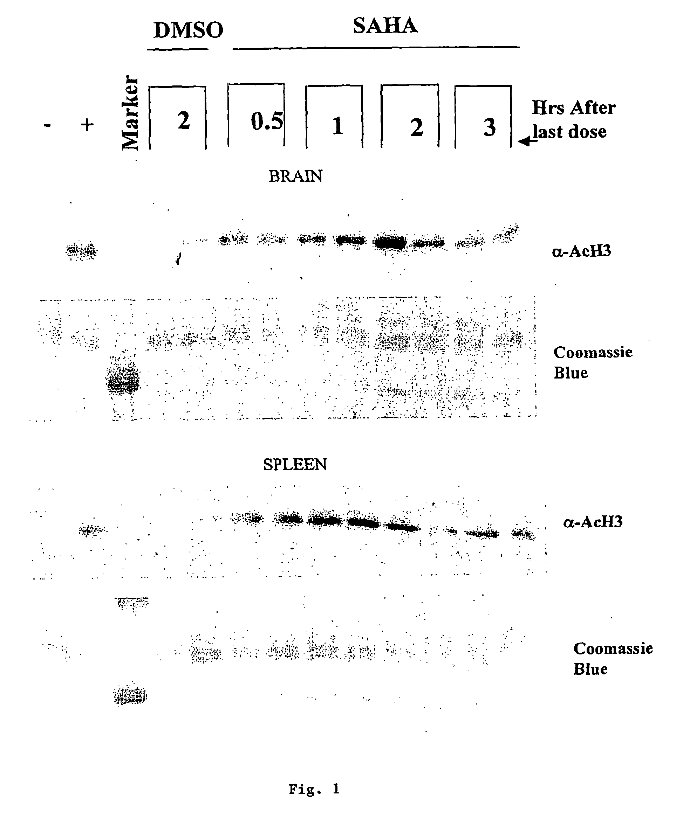

Mice (2 mice per condition) were injected by intrapentoneal injection (IP) with either SAHA (100 mg/kg), pyroxamide (200 mg/kg), or vehicle (dimethylsulfoxide). Each mouse was administered three injections at the indicated dose at 1 hour intervals. After the final IP injection, tissues (brain, spleen or liver) were isolated at the times indicated. Histones were isolated from tissues essentially as described by Yoshida et al., (1990) J. Biol. Chem. 265:17174-17179. Equal amounts of histones (1 µg) were electrophoresed on 15% SDS-polyacrylamide gels and transferred to Hybond-P filters (Amersham). Filters were blocked with 3% milk and probed with a rabbit purified polyclonal anti-acetylated histone H4 antibody (αAc-H4) and anti-acetylated histone H3 antibody (αAc-H3) (Upstate Biotechnology, Inc.). Levels of acetylated histone were visualized using a horseradish peroxidase-conjugated goat anti-rabbit antibody (1:5000) and the SuperSignal chemiluminescent substrate (Pierce). As a loading control for the histone proteins, parallel gels were run and stained with Coomassie Blue (CB). The results are shown in FIGS. 1 and 2.

## Claims

1. Use of suberoylanilide hydroxamic acid (SAHA) represented by the following structure: 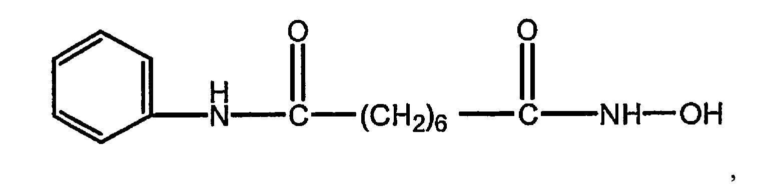 or a pharmaceutically acceptable salt thereof, and a pharmaceutically acceptable carrier or diluent, for the manufacture of an oral medicament for treating brain cancer in a mammal.

2. Use according to Claim 1, wherein SAHA is the active ingredient in the medicament.

3. Use according to Claim 2, wherein the mammal is a human.

4. Use according to Claim 2, wherein the brain cancer is glioma.

5. Use according to Claim 4, wherein the brain cancer is glioblastoma multiforme.

6. Use according to Claim 1, wherein the suberoylanilide hydroxamic acid (SAHA) or pharmaceutically acceptable salt thereof is to be used in combination with a standard therapy agent for the disease being treated.

7. A composition comprising suberoylanilide hydroxamic acid (SAHA) represented by the following structure: 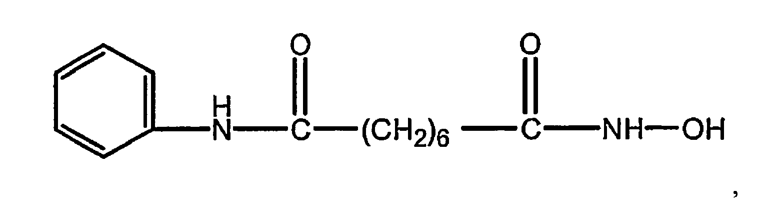 or a pharmaceutically acceptable salt thereof, and a pharmaceutically acceptable carrier or diluent, for use in the treatment of brain cancer in a mammal, wherein the composition is formulated for oral administration.

8. A composition for use according to Claim 7, wherein SAHA is the active ingredient in the composition.

9. A composition for use according to Claim 8, wherein the mammal is a human.

10. A composition for use according to Claim 8, wherein the brain cancer is glioma.

11. A composition for use according to Claim 10, wherein the brain cancer is glioblastoma multiforme.

12. A composition for use according to Claim 7, wherein the composition is for use in combination with a standard therapy agent for the disease being treated.

## Patentansprüche

1. Verwendung von Suberoylanilidhydroxamsäure (SAHA), dargestellt durch die folgende Struktur: 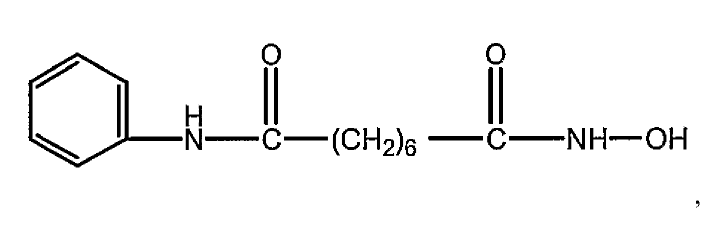 oder eines pharmazeutisch verträglichen Salzes davon und eines pharmazeutisch verträglichen Trägers oder Verdünnungsmittels zur Herstellung eines oralen Medikaments zur Behandlung von Hirnkrebs in einem Säuger.

2. Verwendung gemäß Anspruch 1, wobei SAHA der Wirkstoff in dem Medikament ist.

3. Verwendung gemäß Anspruch 2, wobei der Säuger ein Mensch ist.

4. Verwendung gemäß Anspruch 2, wobei der Hirnkrebs ein Gliom ist.

5. Verwendung gemäß Anspruch 4, wobei der Hirnkrebs ein Glioblastoma multiforme ist.

6. Verwendung gemäß Anspruch 1, wobei die Suberoylanilidhydroxamsäure (SAHA) oder das pharmazeutisch verträgliche Salz davon dazu bestimmt ist, in Kombination mit einem Therapiestandardmittel für die Erkrankung, welche behandelt wird, verwendet zu werden.

7. Zusammensetzung, umfassend Suberoylanilidhydroxamsäure (SAHA), dargestellt durch die folgende Struktur: 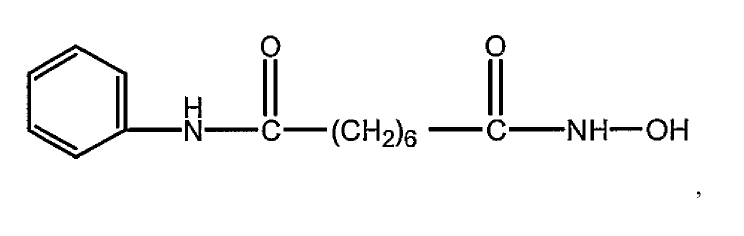 oder ein pharmazeutisch verträgliches Salz davon und einen pharmazeutisch verträglichen Träger oder Verdünnungsmittel, zur Verwendung in der Behandlung von Hirnkrebs in einem Säuger, wobei die Zusammensetzung für orale Verabreichung formuliert ist.

8. Zusammensetzung zur Verwendung gemäß Anspruch 7, wobei SAHA der Wirkstoff in der Zusammensetzung ist.

9. Zusammensetzung zur Verwendung gemäß Anspruch 8, wobei der Säuger ein Mensch ist.

10. Zusammensetzung zur Verwendung gemäß Anspruch 8, wobei der Hirnkrebs ein Gliom ist.

11. Zusammensetzung zur Verwendung gemäß Anspruch 10, wobei der Hirnkrebs ein Glioblastoma multiforme ist.

12. Zusammensetzung zur Verwendung gemäß Anspruch 7, wobei die Zusammensetzung zur Verwendung in Kombination mit einem Therapiestandardmittel für die Erkrankung, welche behandelt wird, ist.

## Revendications

1. Utilisation de l'acide suberoylanilide hydroxamique (SAHA) représenté par la structure suivante : 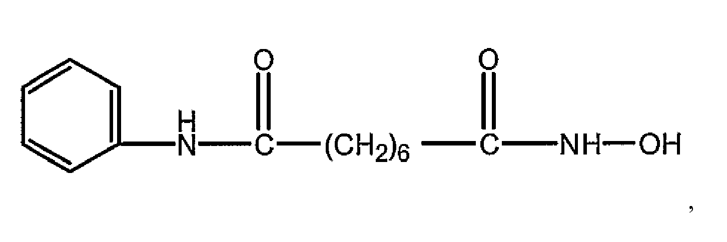 ou d'un sel pharmaceutiquement acceptable de celui-ci, et d'un vecteur pharmaceutiquement acceptable ou d'un diluant, dans la fabrication d'un médicament à administrer par voie orale destiné à traiter le cancer du cerveau chez un mammifère.

2. Utilisation selon la revendication 1, dans laquelle le SAHA est le principe actif dans le médicament.

3. Utilisation selon la revendication 2, dans laquelle le mammifère est un humain.

4. Utilisation selon la revendication 2, dans laquelle le cancer du cerveau est un gliome.

5. Utilisation selon la revendication 4, dans laquelle le cancer du cerveau est un glioblastome multiforme.

6. Utilisation selon la revendication 1, dans laquelle l'acide suberoylanilide hydroxamique (SAHA) ou un sel pharmaceutiquement acceptable de celui-ci doit être utilisé en association avec un agent thérapeutique standard destiné à la maladie traitée.

7. Composition comprenant l'acide suberoylanilide hydroxamique (SAHA) représenté par la structure suivante : 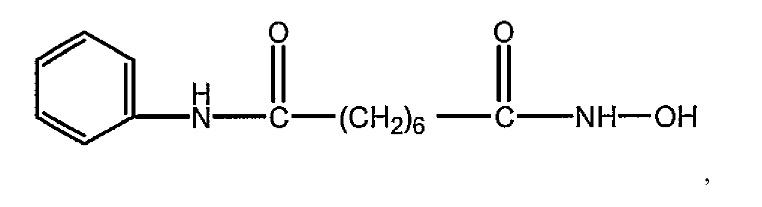 ou un sel pharmaceutiquement acceptable de celui-ci, et un vecteur pharmaceutiquement acceptable ou un diluant, destinée à être utilisée dans le traitement du cancer du cerveau chez un mammifère, la composition étant formulée pour être administrée par voie orale.

8. Composition destinée à être utilisée selon la revendication 7, le SAHA étant le principe actif dans la composition.

9. Composition destinée à être utilisée selon la revendication 8, dans laquelle le mammifère est un humain.

10. Composition destinée à être utilisée selon la revendication 8, dans laquelle le cancer du cerveau est un gliome.

11. Composition destinée à être utilisée selon la revendication 10, dans laquelle le cancer du cerveau est un glioblastome multiforme.

12. Composition destinée à être utilisée selon la revendication 7, la composition devant être utilisée en association avec un agent thérapeutique standard destiné à la maladie traitée.
